Europäisches Patentamt

(19) European Patent Office

Office européen des brevets

(11) Publication number: **0 213 749 B1**

(12) **EUROPEAN PATENT SPECIFICATION**

(45) Date of publication of the patent specification:
11.04.90

(21) Application number: 86305874.9

(22) Date of filing: 30.07.86

(51) Int. Cl.⁴: **A61M 29/02**

(54) **Balloon dilatation catheter with advanceable non-removable guide wire.**

(30) Priority: 30.07.85 US 760397

(43) Date of publication of application:
11.03.87 Bulletin 87/11

(45) Publication of the grant of the patent:
11.04.90 Bulletin 90/15

(84) Designated Contracting States:
AT CH DE FR GB IT LI NL

(56) References cited:
WO-A-84/04686
US-A-3 452 740
US-A-4 411 055

(73) Proprietor: **ADVANCED CARDIOVASCULAR SYSTEMS, INC., 1395 Charleston Road, Mountain View, CA 94039-7101(US)**

(72) Inventor: **Samson, Wilfred Joseph, 19691 Falwell Avenue, Saratoga California 94070(US)**
Inventor: **Frisbie, Jeffrey Steven, 3203 Pine Spring Court, San Jose California 95121(US)**

(74) Representative: **Bizley, Richard Edward, BOULT, WADE & TENNANT 27 Furnival Street, London EC4A 1PQ(GB)**

## Description

This invention relates to a balloon dilatation catheter comprising a flexible tubular member, having first and second lumens extending therethrough, and an inflatable balloon which is carried by the distal portion of the tubular member, the first lumen extending through the balloon and not being in communication with the interior of the balloon, and the second lumen being in communication with the interior of the balloon.

Such a catheter is disclosed in US-A 4 411 055 (Simpson and Robert).

The size of the collapsed balloon profile in balloon dilatation catheters makes it possible to assess the potential ability of the balloon catheter to cross a lesion or stenosis. In general, the smaller the profile of the collapsed balloon, the tighter the lesion (or smaller the hole) the dilatation catheter is able to pass through.

Consequently between two dilatation catheters having the same nominal inflated balloon diameters, the one with the smaller collapsed profile conceivably can cross tighter lesions and thus has a distinct advantage over one with a larger collapsed balloon profile.

In balloon dilatation catheters using removable guide wires, such as disclosed in US-A 4 411 055, the collapsed balloon profile has been reduced by reducing the outside diameter and the inside diameter of the guide wire lumen. This permits the balloon when evacuated and folded around the lumen to have a smaller effective diameter or, in other words, a smaller collapsed balloon profile. However, there is a limitation as to how much reduction in diameter can be obtained with such an approach because it is still necessary to be able to insert and remove the guide wire with such a balloon dilatation catheter. There is therefore a need for a new and improved balloon dilatation catheter with a guide wire in which it is possible to obtain still smaller collapsed balloon profiles.

The present invention is characterised in that a guide wire is slidably disposed in and protrudes from the distal end of the first lumen, the distal end of the guide wire having a coil-like tip with a cross-sectional area which is greater than that of the first lumen and prevents removal of the guide wire through the first lumen.

In the accompanying drawings:

Figure 1 is a side elevational view of a balloon dilatation catheter, and
Figure 2 is an enlarged cross sectional view of a portion of the balloon dilatation catheter shown in Figure 1 which, in particular, shows the construction of the balloon.

As shown in the drawings, a balloon dilatation catheter 10 comprises a flexible elongate tubular member 11 having a first elongate flexible tubular element 12 which is provided with a first lumen 13 extending therethrough. The distal portion of the tubular element 12 is provided with a portion 12a of reduced diameter with a tapered transition portion 12b which adjoins a portion 12c of a larger diameter extending to the proximal extremity of the first tubular element.

A second elongate flexible tubular element 16 is also provided which extends over the first elongate tubular element 12 and is disposed coaxially therewith. The second elongate tubular element 16 is of a size so that there is provided between the first and second tubular elements 12 and 16 an annular lumen 17 extending the length of the tubular elements 12 and 16. A balloon 18 is carried by the distal portion of the tubular element 16 near the distal extremity thereof and has its interior in communication with the lumen 17. The balloon 18 is formed integral with the second tubular element 16.

The first and second tubular elements 12 and 16 can be formed of a suitable flexible thermo-plastic material such as a polyolefin or polyvinylchloride.

The distal extremity of the second elongate tubular element 16 is shrunk onto the distal extremity of the first tubular element 12 to form a liquid-tight seal between their distal ends. The lumen 17 opens into the balloon 18 and can be used for inflating and deflating the balloon. If desired, instead of forming the balloon 18 integral with the second elongate tubular element 16, it can be formed as a separate element and bonded to the second tubular element 16 by suitable means such as an adhesive.

An adapter 21 is secured to the proximal extremity of the tubular member 11. The adapter 21 is of a conventional type and is provided with a main or central arm 22 and a side arm 23. The main arm 22 is in communication with the lumen 13 of the first tubular element 12 and is adapted to carry a guide wire 26. The guide wire 26 can be formed by a suitable material such as stainless steel and is provided with an elongate portion 26a, a necked down tapered portion 26b and a portion 26c at the distal extremity which is of substantially reduced diameter. A spring-like coil 28 is secured to the distal extremity of the guide wire 26 and is in the form of a helix. The proximal end of the coil 28 is secured to the distal portion of the guide wire 26 by solder 29. A semi-spherical tip 31 is provided at the distal extremity of the coil 28 and joins the tip of the wire 26 to the coil. The coil 28 is formed of a suitable material, for example platinum. The tip 31 is formed of a suitable material such as gold.

A band 36, which serves as a marker, is provided on the portion 12a of the first tubular element 12 and is disposed substantially equidistant between the extremities of the balloon 18.

A torquer 41 of a conventional type is secured to the proximal extremity of the guide wire 26 and is adapted to be grasped by hand for rotating the guide wire as hereinafter described.

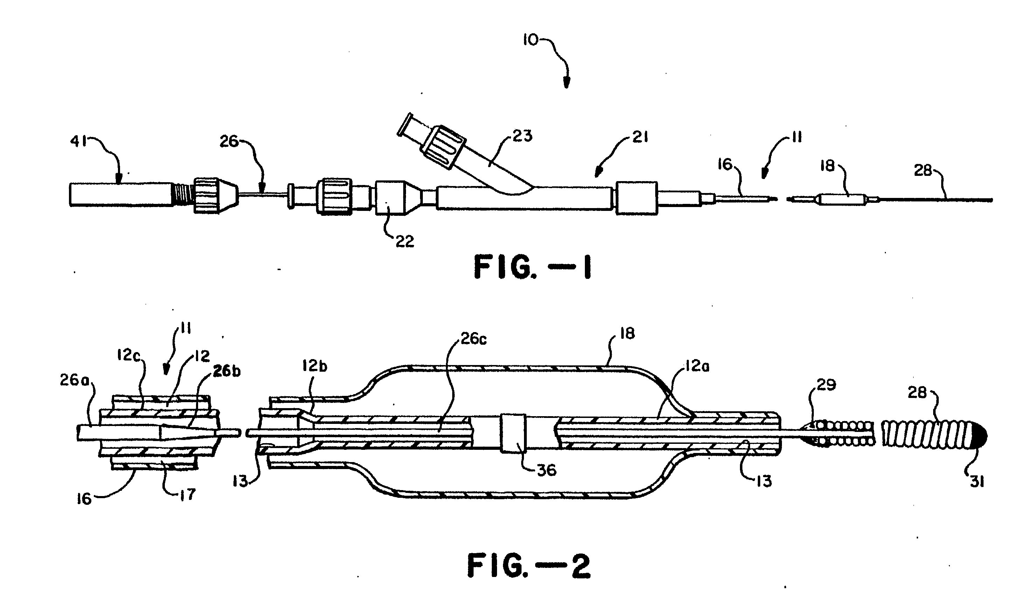

If it is assumed that the balloon 18 has a diameter of 2 mm when fully inflated, the balloon dilatation catheter shown in Figures 1 and 2 of the drawings can have dimensions such as the following: The tubular member 11 extending from the adapter 21 to the balloon 18 can have a suitable length ranging from 40 to 50 cm and preferably a dimension of approximately 135 cm. The portion 12c of the first or inner tubular element 12 can have an outside diameter ranging from 0.064 to 0.86 mm (0.025 to 0.034 inch), an inside diameter of 0.36 to 0.51 mm (0.014 to 0.020 inch) and preferably 0.69 and 0.41 mm (0.027 and 0.016 inch) respectively. The portion 12a can have an outside diameter of 0.41 to 0.56 mm (0.016 to 0.022 inch) and an inside diameter of 0.2 to 0.3 mm (0.008 to 0.012 inch) and preferably dimensions of 0.51 mm (0.020 inch) for the outside diameter and 0.25 mm (0.010 inch) for the inside diameter. The second tubular element 16 can have an outside diameter of 0.97 to 1.21 mm (0.038 to 0.048 inch) and an inside diameter of 0.84 to 1.07 mm (0.033 to 0.042 inch) and preferably an outside diameter of 1.14 mm (0.045 inch) and an inside diameter 0.97 mm (0.038 inch). The balloon 18, 2 mm in size can have a collapsed profile of 0.76 to 0.97 mm (0.030 to 0.038 inch) and can have a suitable length of, for example, 15 to 35 mm and preferably a length of approximately 25 mm. The distal extremity of the catheter 10 in which the distal extremities of the first and second tubular elements 12 and 16 are bonded together can have a suitable dimension ranging from 0.61 to 0.76 mm (0.024 to 0.030 inch) and preferably a dimension of 0.66 mm (0.026 inch). This extremity can have a length ranging from 3 to 10 mm and preferably a length of approximately 5 mm. The portion 26a of the guide wire 26 can have a dimension ranging from 0.33 to 0.36 mm (0.013 to 0.014 inch) and if desired can be coated with a suitable material such as PTFE. The portion 26c of the guide wire 26 can have a dimension such as 0.18 to 0.2 mm (0.007 to 0.008 inch). The coil 28 can have an outside diameter of 0.3 to 0.41 mm (0.012 to 0.016 inch) and preferably of approximately 0.38 mm (0.015 inch). The coil 28 can have a length ranging from 15 mm to 50 mm and preferably has a length of approximately 30 mm. The radiopaque marker band 36 can have an internal diameter of 0.53 mm (0.021 inch) with a wall thickness of 0.05 mm (0.002 inch) and a width of approximately 1.14 mm (0.045 inch).

Operation and use of the balloon dilatation catheter as shown in Figures 1 and 2 is now briefly described as follows. Assume that a stenosis is to be crossed. The catheter 10 with the balloon 18 in a collapsed state is introduced into the vessel in a conventional manner. The guide wire 26 can be utilized to facilitate the introduction and advancement of the dilatation catheter 10. Its progress can be observed upon a fluoroscope because the coil 28 is relatively opaque to X-rays. Although the guide wire 26 cannot be retracted from the catheter, it is independently manipulable with respect to the balloon dilatation catheter. For example, if desired the distal extremity of the guide wire 26 and, in particular, the coil 28 can be advanced so it is 7.6 to 10 cm (3 to 4 inches) ahead of the catheter. It also can be rotated by use of the torquer 41.

By utilizing such a guide wire 26 with the dilatation catheter herein described, it is possible to reduce the profile of the dilatation catheter substantially with the only disadvantage being that the guide wire cannot be removed. The amount of travel of the guide wire 26 is determined by the length of the portion 26c of the guide wire. When the tapered portion 26b enters the tapered portion 12b of the first tubular element 12, further travel is inhibited. The rearmost extremity of the guide wire 26 is determined when the solder 29 reaches the distal extremity of the first flexible tubular element 12. Typically this extendability or advanceability of the guide wire in the dilatation catheter can vary from 10 to 20 cm. The amount of advancement of the guide wire 26 can also be adjusted within the limits just described by the positioning of the torquer 41 on the guide wire.

After the dilatation catheter has been positioned in the stenosis, the balloon 18 can be inflated by introducing a radiopaque contract liquid through the side arm 23. The marker 36 provided within the balloon 18 facilitates the positioning of the balloon in the stenosis prior to inflation. After the balloon 18 has been inflated one or more times, the balloon can be deflated and the balloon removed from the stenosis and the dilatation catheter 10 removed from the vessel.

## Claims

1. A balloon dilatation catheter (10) comprising a flexible tubular member (11), having first and second lumens (13, 17) extending therethrough, and an inflatable balloon (18) which is carried by the distal portion of the tubular member (11), the first lumen (13) extending through the balloon (18) and not being in communication with the interior of the balloon, and the second lumen (17) being in communication with the interior of the balloon (18), characterised in that a guide wire (26) is slidably disposed in and protrudes from the distal end of the first lumen (13), the distal end of the guide wire (26) having a coil-like tip (28) with a cross-sectional area which is greater than that of the first lumen (13) and prevents removal of the guide wire (26) through the first lumen (13).

2. A catheter as claimed in claim 1, characterised in that means (41) is secured to the proximal end of the guide wire (26) to facilitate rotation of the guide wire in the first lumen (13).

3. A catheter as claimed in claim 1 or 2, characterised in that the flexible tubular member (11) comprises a first tubular element (12) through which extends the first lumen (13) and a second tubular element (16) which extends co-axially over the first element (12), the first and second tubular elements forming the second lumen (17), the inflatable balloon (18) being carried by the second tubular element (16).

## Patentansprüche

1. Ballondilatationskatheter (10) mit einem flexiblen rohrförmigen Element (11), durch das ein erster und ein zweiter lichter Raum (13, 17) verläuft, und mit einem vom freien Teil des rohrförmigen Elementes (10) getragenen aufblasbaren Ballon (18), wobei sich der erste lichte Raum (13) durch den Ballon (18) erstreckt und mit dessen Innerem nicht in Verbindung steht und der zweite lichte Raum (17) mit dem Inneren des Ballons (18) in Verbindung steht, dadurch gekennzeichnet, daß im freien Ende des ersten lichten Raums (13) ein Führungsdraht (26) gleitend angeordnet ist und aus diesem herausragt, dessen freies Ende eine spulenförmige Spitze (28) mit einem Querschnitt besitzt, der größer als der des ersten lichten Raumes (13) ist und ein Entfernen des Führungsdrahtes (26) durch den ersten lichten Raum verhindert.

2. Katheter nach Anspruch 1, dadurch gekennzeichnet, daß zur Erleichterung der Drehung des Führungsdrahtes im ersten lichten Raum (13) Mittel (41) am benachbarten Ende des Führungsdrahtes (26) befestigt sind.

3. Katheter nach Anspruch 1 oder 2, dadurch gekennzeichnet, daß das flexible rohrförmige Element (11) ein erstes rohrförmiges Element (12), durch das sich der erste lichte Raum (13) erstreckt, und ein zweites rohrförmiges Element (16), das sich koaxial über das erste Element (12) erstreckt, umfaßt, daß das erste und zweite rohrförmige Element den zweiten lichten Raum (17) bilden und daß der aufblasbare Ballon (18) vom zweiten rohrförmigen Element (16) getragen ist.

**Revendications**

1. Sonde de dilatation à ballonnet (10) comportant un organe tubulaire flexible (11), présentant une première et une deuxième lumières (13, 17) s'étendant à l'intérieur de l'organe sur toute sa longueur, et un ballonnet gonflable (18) porté par la partie distale de l'organe tubulaire (11), la première lumière (13) s'étendant à travers le ballonnet (18) et n'étant pas en communication avec l'intérieur du ballonnet, et la deuxième lumière (17) étant en communication avec l'intérieur du ballonnet (18), caractérisée en ce qu'un câble de guidage (26) est placé de manière coulissante dans la première lumière (13) et s'étend au-delà de l'extrémité distale de la première lumière, l'extrémité distale du câble de guidage (26) présentant un bout en forme de ressort (28 dont le profil est supérieur à celui de la première lumière (13) et qui empêche le câble de guidage (26) de sortir à travers la première lumière (13).

2. Sonde selon la revendication 1, caractérisée en ce que le moyen (41) est fixé à l'extrémité proximale du câble de guidage (26) pour faciliter la rotation du câble de guidage dans la première lumière (13).

3. Sonde selon la revendication 1 ou 2, caractérisée en ce que l'organe tubulaire flexible (11) comporte un premier élément tubulaire (12) à travers lequel s'étend la première lumière (13) et un deuxième élément tubulaire (16) s'étendant coaxialement par-dessus le premier élément (12), le premier et le deuxième éléments tubulaires formant la deuxième lumière (17), le ballonnet gonflable (18) étant porté par le deuxième élément tubulaire (16).

FIG.—1

FIG.—2